# EUROPEAN PATENT SPECIFICATION

(11) **EP 1 554 977 B1**
(45) Date of publication and mention of the grant of the patent: **02.05.2007**
(21) Application number: 05000080.1
(22) Date of filing: 04.01.2005
(51) Int. Cl.: A61B 5/053, A61B 5/22

(54) **Impedance based muscular strengh measuring device**
Vorrichtung zur Messung der Muskelkraft mittels Impedanz
Dispositif de mesure de force musculaire à base d'impédance

(30) Priority: 16.01.2004 JP 2004008626
(43) Date of publication of application: 20.07.2005
(73) Proprietor: TANITA CORPORATION, Tokyo (JP)
(72) Inventor: Izumi, Shuichi, Tokyo (JP)
(74) Representative: Grünecker, Kinkeldey, Stockmair & Schwanhäusser Anwaltssozietät

(56) References cited:
- EP-A- 1 306 051
- DATABASE MEDLINE [Online] US NATIONAL LIBRARY OF MEDICINE (NLM), BETHESDA, MD, US; May 2003 (2003-05), GOSKER HARRY R ET AL: "Striking similarities in systemic factors contributing to decreased exercise capacity in patients with severe chronic heart failure or COPD." XP002322192 Database accession no. NLM12740256 & CHEST. MAY 2003, vol. 123, no. 5, May 2003 (2003-05), pages 1416-1424, ISSN: 0012-3692
- DATABASE MEDLINE [Online] US NATIONAL LIBRARY OF MEDICINE (NLM), BETHESDA, MD, US; July 2001 (2001-07), MIYATANI M ET AL: "Validity of estimating limb muscle volume by bioelectrical impedance." XP002322193 Database accession no. NLM11408456 & JOURNAL OF APPLIED PHYSIOLOGY (BETHESDA, MD. : 1985) JUL 2001, vol. 91, no. 1, July 2001 (2001-07), pages 386-394, ISSN: 8750-7587

## Description

### BACKGROUND OF THE INVENTION

### (i) Field of the Invention

This invention relates to a measuring device which determines muscular strength by measuring impedance.

### (ii) Description of the Related Art

Conventional muscular strength measuring devices determine muscular strength by detecting force produced by moving a specific body part (muscle) by use of a force sensor (sensor for detecting external force). For example, a "grip dynamometer" disclosed in Patent Publication 1 measures the muscular strength of a muscle related to gripping by detecting force produced when a subject grips an immovable member and a movable member by use of a sensor (force sensor) . Further, a "quadriceps femoris muscle strength measuring device" disclosed in Patent Publication 2 measures the muscular strength of a quadriceps femoris muscle by detecting the pressing force of the back of a knee against a knee backside support when a leg is stretched with the back of the knee of the leg supported, by use of a strain gauge load sensor (force sensor) .

### Patent Publication 1

Japanese Patent Laid-Open Publication No. 9-313467

### Patent Publication 2

Japanese Patent Laid-Open Publication No. 11-290301

However, the above measuring devices which determine muscular strength by use of a force sensor have a relatively complex, strong structural form due to having a mechanism for passing force produced by moving a specific body part to the force sensor accurately and preventing the force produced by moving the specific body part from causing breakages. Therefore, they have a problem of increases in production processes and costs.

Further, they have a problem of limited capability of determining only the muscular strength of a limited specific body part, as exemplified by only the muscular strength of a muscle related to gripping or only the muscular strength of a quadriceps femoris muscle.

Thus, in view of the above problems, an object of the present invention is to provide an impedance based muscular strength measuring device which can achieve less production processes and costs than the prior art. Another object of the present invention is to provide an impedance based muscular strength measuring device which can also determine muscular strength in a plurality of specific body parts

### SUMMARY OF THE INVENTION

An impedance based muscular strength measuring device of the present invention comprises:
muscle relaxed state directing means,
muscle tensed state directing means,
impedance measuring means,
impedance change rate computing means,
muscular strength regression formula storing means, and
muscular strength computing means,
wherein
the muscle relaxed state directing means gives a direction that a muscle in a specific body part is to be relaxed,
the muscle tensed state directing means gives a direction that the muscle in the specific body part is to be tensed,
the impedance measuring means has an electrode set to make contact with the specific body part and supplies a current to the specific body part and detects a voltage by use of the electrode set to measure the impedance of the relaxed muscle in the specific body part after the muscle relaxed state directing means gives the direction and the impedance of the tensed muscle in the specific body part after the muscle tensed state directing means gives the direction,
the impedance change rate computing means computes the rate of change in impedance between the impedance of the relaxed muscle and the impedance of the tensed muscle which are measured by the impedance measuring means,
the muscular strength regression formula storing means stores a muscular strength regression formula which represents a correlation between the rate of change in the impedance of the muscle in the specific body part and the muscular strength of the specific body part, and
the muscular strength computing means computes the muscular strength of the specific body part by substituting the rate of change in impedance which is computed by the impedance change rate computing means into the muscular strength regression formula stored in the muscular strength regression formula storing means.

Further, the electrode set of the impedance measuring means is movably contacted with each of specific body parts, the muscular strength regression formula storing means stores a plurality of muscular strength regression formulae corresponding to the respective specific body parts and comprises muscle selecting/inputting means for selecting and inputting a muscle in a specific body part to be measured and muscular strength regression formula selecting means for selecting a muscular strength regression formula corresponding to the specific body part to be measured which is selected and input by the muscle selecting/inputting means from the muscular strength regression formulae stored in the muscular strength regression formula storing means, and the muscular strength computing means computes the muscular strength of the specific body part by substituting the rate of change in impedance which is computed by the impedance change rate computing means into the muscular strength regression formula selected by the muscular strength regression formula selecting means.

The impedance based muscular strength measuring device of the present invention clearly notifies a subject that a muscle in a specific body part is to be relaxed or tensed by the muscle relaxed state directing means and the muscle tensed state directing means, measures impedance with the electrode set in contact with the specific body part by the impedance measuring means, and determines the muscular strength of the specific body part through computations performed by the impedance change rate computing means and the muscular strength computing means. Accordingly, the impedance based muscular strength measuring device of the present invention can adopt a relatively simple structural form, thereby reducing production processes and costs.

Further, the electrode set of the impedance measuring means is contacted with each of specific body parts, a specific body part to be measured is selected and input by the muscle selecting/inputting means, a muscular strength regression formula corresponding to the selected specific body part is selected by the muscular strength regression formula selecting means, and the muscular strength of the specific body part is determined based on the selected muscular strength regression formula by the muscular strength computing means. Thus, the muscular strength of each of specific body parts can be determined.

### BRIEF DESCRIPTION OF THE DRAWINGS

Fig. 1 is an external view of an impedance based muscular strength measuring device as an example of the present invention.
Fig. 2 is a block diagram illustrating the overall constitution of the device of Fig. 1.
Fig. 3 is a flowchart illustrating the application mode and operational procedures of the device of Fig. 1.
Fig. 4 is a diagram illustrating a graph of the relationship between grip strength and an impedance change rate.

### DETAILED DESCRIPTION OF THE PREFERRED EMBODIMENTS

An impedance based muscular strength measuring device of the present invention comprises muscle relaxed state directing means, muscle tensed state directing means, impedance measuring means, impedance change rate computing means, muscular strength regression formula selecting means, muscular strength regression formula storing means, and muscular strength computing means.

A muscle selecting/inputting means selects and inputs a muscle in a specific body part to be measured from muscles in a number of specific body parts. The muscle relaxed state directing means gives a direction that the muscle in the specific body part to be measured is to be relaxed. The muscle tensed state directing means gives a direction that the muscle in the specific body part to be measured is to be tensed. The impedance measuring means has an electrode set which is movably contacted with each of specific body parts and supplies a current to the specific body part and detects a voltage by use of the electrode set to measure the impedance of the relaxed muscle in the specific body part after the muscle relaxed state directing means gives the above direction and the impedance of the tensed muscle in the specific body part after the muscle tensed state directing means gives the above direction. The impedance change rate computing means computes the rate of change in impedance between the impedance of the relaxed muscle and the impedance of the tensed muscle which are measured by the impedance measuring means. The muscular strength regression formula storing means stores a muscular strength regression formula which represents a correlation between the rate of change in the impedance of a muscle in a specific body part and the muscular strength of the specific body part for each of specific body parts. The muscular strength regression formula selecting means selects a muscular strength regression formula corresponding to the muscle in the specific body part to be measured which is selected by the muscle selecting/inputting means from the muscular strength regression formulae stored in the muscular strength regression formula storing means. The muscular strength computing means computes the muscular strength of the specific body part by substituting the rate of change in impedance which is computed by the impedance change rate computing means into the muscular strength regression formula stored in the muscular strength regression formula storing means and selected by the muscular strength regression formula selecting means.

The "relaxed state" refers to a muscle under normal tension. The "tensed state" refers to a muscle under tension different from the normal tension or a contracted muscle.

Hereinafter, the impedance based muscular strength measuring device of the present invention will be further described with reference to an example.

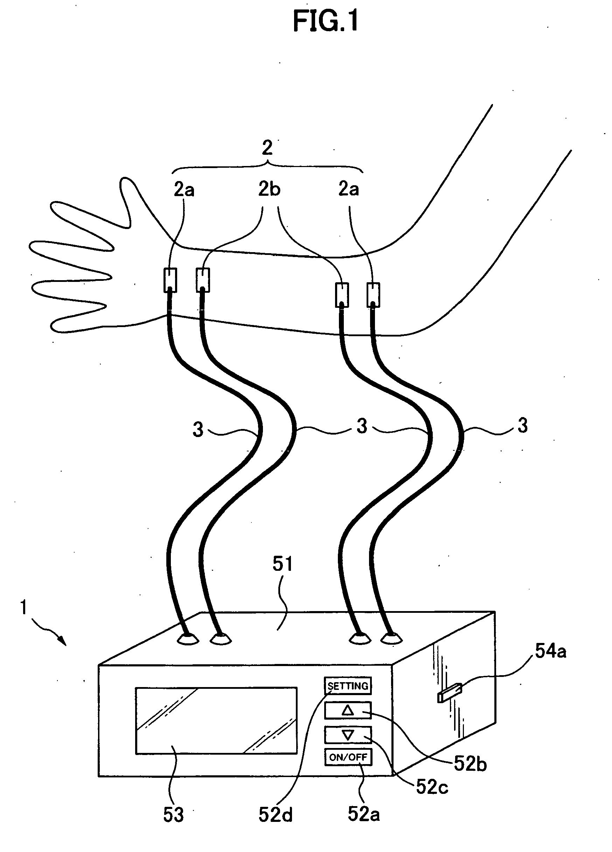

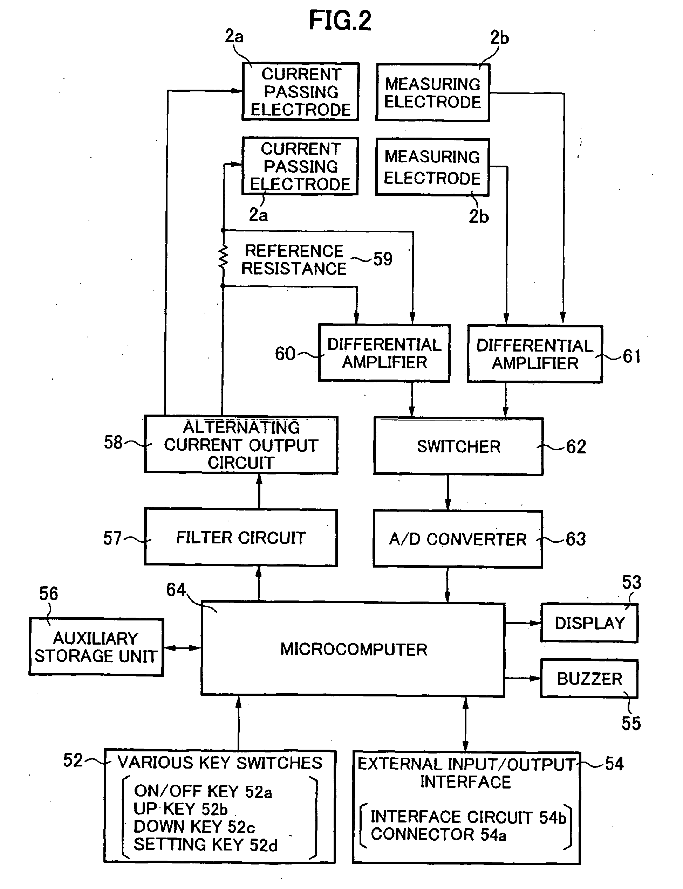

First, a specific constitution of the impedance based muscular strength measuring device of the present invention will be described by use of an external view shown in Fig. 1 and a block diagram shown in Fig. 2.

The impedance based muscular strength measuring device of the present invention roughly comprises a main unit 1, an electrode set 2, and cords 3 which connect the electrode set 2 to the main unit 1, as shown in Fig. 1.

The electrode set 2 comprises current passing electrodes 2a for passing a current through a specific body part and measuring electrodes 2b for detecting a voltage occurring during energization of a specific body part. The electrodes can be detachably attached to a specific body part.

Further, the number of the cords 3 corresponds to the number of electrodes in the electrode set 2. One end of the cord 3 is connected to the electrode 2a or 2b, and the other end thereof is connected to a circuit line of an electronic circuit card unit.

Further, the main unit 1 incorporates the electronic circuit card unit in a case 51 and has various key switches (ON/OFF key 52a, UP key 52b, DOWN key 52c, setting key 52d), a display 53, and a connector 54a which constitutes a portion of an external input/output interface 54, on the external surface of the case 51.

The electronic circuit card unit, as shown in Fig. 2, comprises an interface circuit 54b constituting a portion of the external input/output interface 54, a buzzer 55, an auxiliary storage unit 56, a filter circuit 57, an alternating current output circuit 58, a reference resistance 59, differential amplifiers 60 and 61, a switcher 62, an A/D converter 63, and a microcomputer 64.

The display 53 displays data associated with selection of a muscle in a specific body part to be measured, a direction of a muscle relaxed state, a direction of a muscle tensed state, the result of muscular strength and the like.

The ON/OFF key 52a is used to turn on or turn off the present device.

The UP key 52b and the DOWN key 52c are used to move a cursor at the time of input.

The setting key 52d is used to set a cursor position selected by the UP key 52b and the DOWN key 52c.

The connector 54a serves as a port to exchange data with an external device.

The interface circuit 54b exchanges signals with an external device via the connector 54a.

The buzzer 55 makes a buzzer sound as a direction of a muscle tensed state.

The auxiliary storage unit 56 stores selected and input data of a specific body part to be measured in an updatable manner.

The filter circuit 57 forms a signal output from the microcomputer 64 into a signal for energization.

The alternating current output circuit 58 obtains a certain execution value from a signal output from the filter circuit 57.

The reference resistance 59 is a resistance (impedance) which is connected to one output terminal of the alternating current output circuit 58 and serves as a reference for correcting the influence of change in a constant current from the alternating current output circuit 58 on impedance.

The differential amplifier 60 amplifies voltages occurring on both sides of the reference resistance 59. The differential amplifier 61 amplifies voltages detected by the measuring electrodes 2b.

The switcher 62 selects and outputs an output from the differential amplifier 60 or an output from the differential amplifier 61 under the control of the microcomputer 64.

The A/D converter 63 converts an analog signal which is an output from the switcher 62 to a digital signal and outputs the digital signal to the microcomputer.

The microcomputer 64 comprises a CPU, a ROM that stores programs for control and computation, selections of muscles in specific body parts, muscular strength regression formulae for computing muscular strengths in specific body parts and direction data of a muscle relaxed state and a muscle tensed state, a RAM which temporarily stores computation results, programs read from external sources and selected/input data on a specific body part to be measured, a timer and an I/O port. The microcomputer 64 executes processes such as selection and input of a muscle in a specific body part to be measured, measurement of impedance of a specific body part in a muscle relaxed state or muscle tensed state, a direction of a muscle relaxed state or muscle tensed state, computations of the rate of change in impedance (impedance change rate), muscular strength and the like, and control relating to display of the result of muscular strength. The selections of muscles in specific body parts are a muscle related to gripping, a rectus abdominis muscle, a quadriceps femoris muscle and a biceps brachii muscle. Further, a muscular strength regression formula for computing the muscular strength of a specific body part represents a correlation between the rate of change in the impedance of the muscle in the specific body part and the muscular strength of the specific body part. The muscular strength regression formulae for computing muscular strengths in specific body parts are Y₁ = a₁X - b₁ for muscular strength of the muscle related to gripping, Y₂ = a₂X - b₂ for muscular strength of the rectus abdominis muscle, Y₃ = a₃X - b₃ for muscular strength of the quadriceps femoris muscle and Y₄ = a₄X - b₄ for muscular strength of the biceps brachii muscle (wherein X represents an impedance change rate, and a₁ to a₄ and b₁ to b₄ represent coefficients (constants)). As the direction of the muscle relaxed state, a message "Start a measurement." is displayed, and as the direction of the muscle tensed state, a message "STRAIN YOUR MUSCLE. " is displayed and a buzzer sound is emitted.

The UP key 52b, DOWN key 52c, setting key 52d, display 53, auxiliary storage unit 56 and microcomputer 64 constitute muscle selecting/inputting means. The display 53 and microcomputer 64 constitute muscle relaxed state directing means. The buzzer 55, display 53 and microcomputer 64 constitute muscle tensed state directing means. The electrode set 2, cords 3, filter circuit 57, alternating current output circuit 58, reference resistance 59, differential amplifiers 60 and 61, switcher 62, A/D converter 63 and microcomputer 64 constitute impedance measuring means. The microcomputer 64 constitutes impedance change rate computing means, muscular strength regression formula storing means, muscular strength regression formula selecting means and muscular strength computing means.

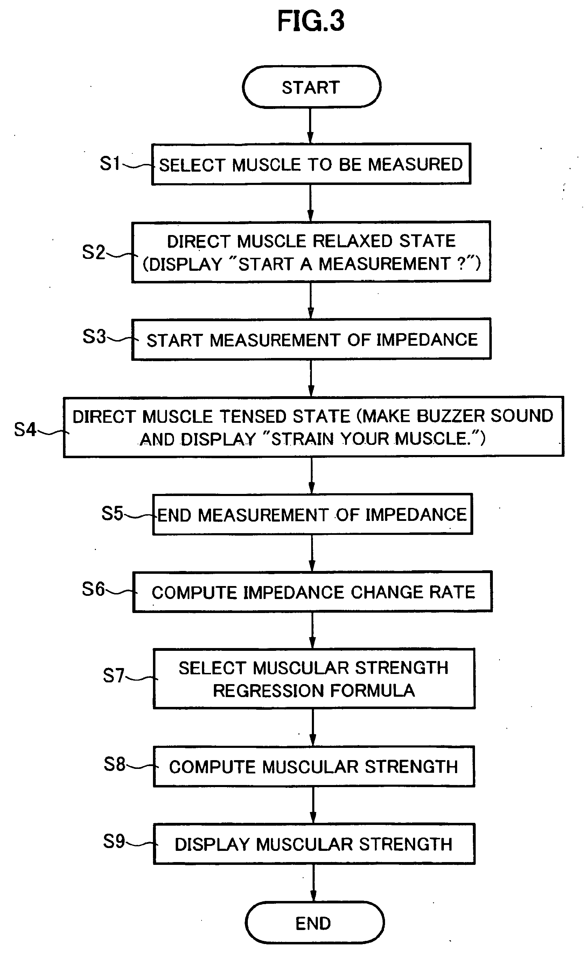

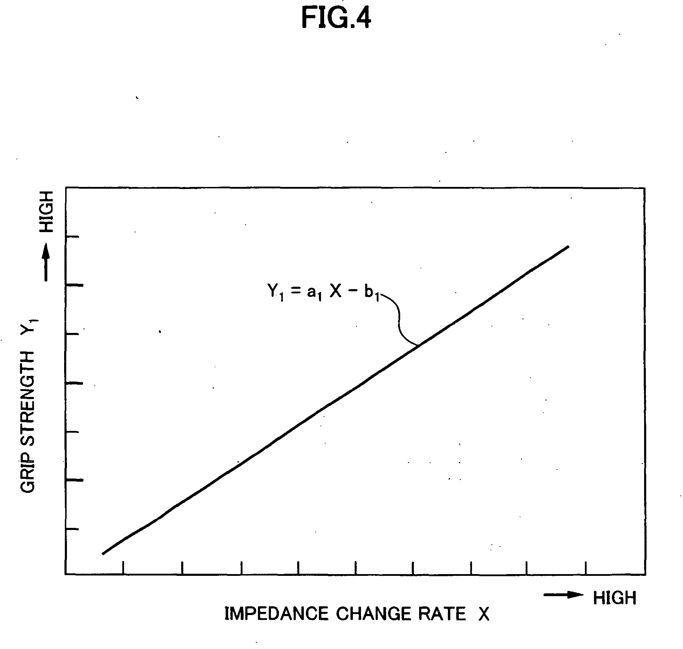

Next, an application mode, functions and operations of an impedance based muscular strength measuring device having the above constitution of the impedance based muscular strength measuring device of the present invention will be described by use of a flowchart shown in Fig. 3 and a diagram shown in Fig. 4 showing the relationship between muscular strength (grip strength) and the rate of change in impedance (impedance change rate).

First, when the ON/OFF key 52a of the case 51 of the main unit 1 is pressed, the components of the device are activated, and the microcomputer 64 displays selections (muscle related to gripping, rectus abdominis muscle, quadriceps femoris muscle and biceps brachii muscle) of muscles in specific body parts on the display 53. Then, a user selects and inputs a desired muscle in a specific body part to be measured (e.g., muscle related to gripping) from the selections (muscle related to gripping, rectus abdominis muscle, quadriceps femoris muscle, and biceps brachii muscle) of muscles in specific body parts which are displayed on the display 53 by use of the UP key 52b, DOWN key 52c and setting key 52d. More specifically, at the press of the UP key 52b or DOWN key 52c, a cursor portion indicating a muscle (e.g., biceps brachii muscle) in a specific body part out of the selections (muscle related to gripping, rectus abdominis muscle, quadriceps femoris muscle, and biceps brachii muscle) of muscles in specific body parts which are displayed on the display 53 moves to a muscle (e.g., muscle related to gripping) in another specific body part. When the setting key 52d is pressed at this point, the microcomputer 64 sets the muscle (muscle related to gripping) in the specific body part indicated by the cursor portion and stores the set data in the auxiliary storage unit 56 (STEP S1).

Then, a message "Start a measurement ?" is displayed on the display 53 as a direction of a muscle relaxed state to a subject (STEP S2).

Then, as shown in Fig. 1, the electrodes are attached to the desired specific body part (forearm) to be measured of the subject (refer to Fig. 1), and the relaxed state of the muscle (muscle related to gripping) is maintained. At the press of the setting key 52d, the microcomputer 64 starts measurement of the impedance of the specific body part. Thereby, the device acquires the impedance of the relaxed muscle in the specific body part. More specifically, the device passes a current through the specific body part (forearm) of the subject from the current passing electrodes 2a based on a signal output from the microcomputer 64 and temporarily stores, as the impedance of the relaxed muscle in the specific body part, impedance based on a signal level based on a voltage occurring in the specific body part and detected by the measuring electrodes 2b, in the RAM of the microcomputer 64 (STEP S3) .

Then, as a direction of a muscle tensed state to the subject, a buzzer sound is emitted from the buzzer 55, and a message "STRAIN YOUR MUSCLE." is displayed on the display 53 (STEP S4).

Then, the microcomputer 64 acquires the impedance of the tensed muscle in the specific body part. More specifically, after the direction of the muscle tensed state, the microcomputer 64 temporarily stores, as the impedance of the tensed muscle in the specific body part, impedance based on the maximum signal level in the RAM of the microcomputer 64, when receiving the maximum signal level higher than or equal to the signal level based on the voltage occurring in the specific body part (forearm) in the relaxed state. Then, the microcomputer 64 ends the measurement of the impedance of the specific body part (STEP S5).

Then, the microcomputer 64 computes an impedance change rate. More specifically, the microcomputer 64 computes, as an impedance change rate (ΔZ), a value obtained by dividing the impedance (Z₁) of the specific body part in the tensed state by the impedance (Z₀) of the specific body part in the relaxed state which are stored temporarily in the RAM of the microcomputer 64, i.e., Z₁/Z₀, and stores it in the RAM temporarily (STEP S6).

Then, the microcomputer 64 selects a muscular strength regression formula corresponding to the selected muscle (muscle related to gripping) in the specific body part which is stored in the auxiliary storage unit 56 from muscular strength regression formulae (regression formula for muscular strength of the muscle related to gripping, regression formula for muscular strength of the rectus abdominis muscle, regression formula for muscular strength of the quadriceps femoris muscle and regression formula for muscular strength of the biceps brachii muscle) for computing muscular strength in the specific body parts which are stored in the ROM.

Thereafter, the microcomputer 64 computes the muscular strength Y₁ of the muscle related to gripping by substituting the impedance change rate (ΔZ) which is stored temporarily in the RAM into a variable X in the selected muscular strength regression formula (regression formula for muscular strength of the muscle related to gripping) Y₁ = a₁X - b₁ (STEP S8) .

Subsequently, the microcomputer 64 displays the computed muscular strength of the muscle related to gripping on the display 53 (STEP S9) . The whole operation of the device is completed through a sequence of steps as described above.

In the above example, an impedance change rate (ΔZ = Z₁/Z₀) is computed as the rate of change in impedance. However, for example, it is also practicable that a value obtained by dividing a difference between the impedance (Z₁) of a specific body part in a tensed state and the impedance (Z₀) of the specific body part in a relaxed state by the impedance (Z₀) of the specific body part in the relaxed state, i.e., I (Z₁-Z₀)|/Z₀, is computed as the amount of change in impedance (ΔZ).

Further, in the above example, a muscle in a specific body part is selected from muscles in a plurality of specific body parts, and a measurement is made. However, it is also practicable that only a muscle in one specific body part is measured without making a selection. In this case, the following constitution is satisfactory. That is, the muscle selecting/inputting means and the muscular strength regression formula selecting means are omitted, the electrode set of the impedance measuring means is connectable to only one specific body part, the muscular strength regression formula storing means stores a muscular strength regression formula for the muscle in the specific body part, and the muscular strength computing means substitutes the rate of change in impedance which is computed by the impedance change rate computing means into the muscular strength regression formula for the muscle in the specific body part.

## Claims

1. An impedance based muscular strength measuring device comprising:
muscle relaxed state directing means,
muscle tensed state directing means,
impedance measuring means,
impedance change rate computing means,
muscular strength regression formula storing means, and
muscular strength computing means,
wherein
the muscle relaxed state directing means gives a direction that a muscle in a specific body part is to be relaxed,
the muscle tensed state directing means gives a direction that the muscle in the specific body part is to be tensed,
the impedance measuring means has an electrode set to make contact with the specific body part and supplies a current to the specific body part and detects a voltage by use of the electrode set to measure the impedance of the relaxed muscle in the specific body part after the muscle relaxed state directing means gives the direction and the impedance of the tensed muscle in the specific body part after the muscle tensed state directing means gives the direction,
the impedance change rate computing means computes the rate of change in impedance between the impedance of the relaxed muscle and the impedance of the tensed muscle which are measured by the impedance measuring means,
the muscular strength regression formula storing means stores a muscular strength regression formula which represents a correlation between the rate of change in the impedance of the muscle in the specific body part and the muscular strength of the specific body part, and
the muscular strength computing means computes the muscular strength of the specific body part by substituting the rate of change in impedance which is computed by the impedance change rate computing means into the muscular strength regression formula stored in the muscular strength regression formula storing means.

2. The device of claim 1, wherein
the electrode set of the impedance measuring means is movably contacted with each of specific body parts,
the muscular strength regression formula storing means stores a plurality of muscular strength regression formulae corresponding to the respective specific body parts and comprises muscle selecting/inputting means for selecting and inputting a muscle in a specific body part to be measured and muscular strength regression formula selecting means for selecting a muscular strength regression formula corresponding to the specific body part to be measured which is selected and input by the muscle selecting/inputting means from the muscular strength regression formulae stored in the muscular strength regression formula storing means, and
the muscular strength computing means computes the muscular strength of the specific body part by substituting the rate of change in impedance which is computed by the impedance change rate computing means into the muscular strength regression formula selected by the muscular strength regression formula selecting means.

3. The device of claim 2, wherein the muscular strength regression formulae are formulae expressed as Y₁ = a₁X - b₁, Y₂ = a₂X - b₂, Y₃ = a₃X - b₃ and Y₄ = a₄X - b₄ wherein Y₁ represents the muscular strength of a muscle related to gripping, Y₂ represents the muscular strength of a rectus abdominis muscle, Y₃ represents the muscular strength of a quadriceps femoris muscle, Y4 represents the muscular strength of a biceps brachii muscle, X represents the rate of change in impedance, and a₁ to a₄ and b₁ to b₄ represent coefficients, and
the muscle in the specific body part to be measured is a muscle related to gripping, a rectus abdominis muscle, a quadriceps femoris muscle or a biceps brachii muscle.

4. The device of any one of claims 1 to 3, wherein the rate of change in impedance is a value obtained by dividing the impedance of the specific body part in a tensed state by the impedance of the specific body part in a relaxed state.

5. The device of any one of claims 1 to 3, wherein the rate of change in impedance is a value obtained by dividing the difference between the impedance of the specific body part in a tensed state and the impedance of the specific body part in a relaxed state by the impedance of the specific body part in a relaxed state.

## Patentansprüche

1. Vorrichtung zum Messen von Muskelstärke auf Basis von Impedanz, die umfasst:
ein Einrichtung zum Anweisen eines entspannten Muskelzustandes,
ein Einrichtung zum Anweisen eines gespannten Muskelzustandes,
eine Impedanzmesseinrichtung,
ein Einrichtung zum Berechnen einer Impedanz-Änderungsrate,
eine Einrichtung zum Speichern einer Muskelstärke-Regressionsformel, und
eine Muskelstärke-Berechnungseinrichtung,
wobei
die Einrichtung zum Anweisen eines entspannten Muskelzustandes eine Anweisung gibt, dass ein Muskel in einem bestimmten Körperteil zu spannen ist,
die Einrichtung zum Anweisen eines gespannten Muskelzustandes eine Anweisung gibt, dass der Muskel in dem bestimmten Körperteil zu entspannen ist,
die Impedanzmesseinrichtung einen Elektrodensatz zum Herstellen von Kontakt mit dem bestimmten Körperteil aufweist, dem bestimmten Körperteil einen Strom zuführt und eine Spannung durch den Einsatz des Elektrodensatzes erfasst, um, nachdem die Einrichtung zum Anweisen eines entspannten Muskelzustandes die Anweisung gibt, die Impedanz des entspannten Muskels in dem bestimmten Körperteil zu messen und, nachdem die Einrichtung zum Anweisen eines gespannten Muskelzustandes die Anweisung gibt, die Impedanz des gespannten Muskels in dem bestimmten Körperteil,
die Einrichtung zum Berechnen einer Impedanz-Änderungsrate die Rate der Änderung der Impedanz zwischen der Impedanz des entspannten Muskels und der Impedanz des gespannten Muskels, die von der Impedanzmesseinrichtung gemessen werden, berechnet,
die Einrichtung zum Speichern einer Muskelstärke-Regressionsformel eine Muskelstärke-Regressionsformel speichert, die eine Korrelation zwischen der Rate der Änderung der Impedanz des Muskels in dem bestimmten Körperteil und der Muskelstärke des bestimmten Körperteils darstellt, und
die Muskelstärke-Berechnungseinrichtung die Muskelstärke des bestimmten Körperteils berechnet, indem sie die Rate der Änderung der Impedanz, die von der Einrichtung zum Berechnen der Impedanz-Änderungsrate berechnet wird, in die Muskelstärke-Regressionsformel einsetzt, die in der Einrichtung zum Speichern der Muskelstärke-Regressionsformel gespeichert ist.

2. Vorrichtung nach Anspruch 1, wobei
der Elektrodensatz der Impedanzmesseinrichtung beweglich mit jedem bestimmter Körperteile in Kontakt gebracht wird,
die Einrichtung zum Speichern der Muskelstärke-Regressionsformel eine Vielzahl von Muskelstärke-Regressionsformeln speichert, die den jeweiligen bestimmten Körperteilen entsprechen, und eine Einrichtung zum Auswählen /Eingeben von Muskeln, mit der ein zumessender Muskel in einem bestimmten Körperteil ausgewählt und eingegeben wird, sowie eine Einrichtung zum Auswählen einer Muskelstärke-Regressionsformel umfasst, mit der eine Muskelstärke-Regressionsformel, die dem zu messenden bestimmten Körperteil entspricht, der durch die Einrichtung zum Auswählen /Eingeben eines Muskels ausgewählt und eingegeben wird, aus den in der Einrichtung zum Speichern der Muskelstärke-Regressionsformel gespeicherten Muskelstärke-Regressionsformeln ausgewählt wird, und
die Muskelstärke-Berechnungseinrichtung die Muskelstärke des bestimmten Körperteils berechnet, indem sie die Rate der Änderung der Impedanz, die von der Einrichtung zum Berechnen der Impedanz-Änderungsrate berechnet wird, in die von der Einrichtung zum Auswählen einer Muskelstärke-Regressionsformel ausgewählten Muskelstärke-Regressionsformel einsetzt.

3. Vorrichtung nach Anspruch 2, wobei die Muskelstärke-Regressionsformeln Formeln sind, die als Y₁ = a₁X - b₁, Y₂ = a₂X - b₂, Y₃ = a₃X - b₃ und Y₄ = a₄X - b₄ ausgedrückt werden, wobei Y₁ die Muskelstärke eines Muskels bezüglich des Greifens darstellt, Y₂ die Muskelstärke eines Rectus-Abdominis-Muskels darstellt, Y₃ die Muskelstärke eines Quadrizeps-Femoris-Muskels darstellt, Y₄ die Muskelstärke eines Bizeps-Brachii-Muskels darstellt, X die Rate der Änderung der Impedanz darstellt und a₁ bis a₄ sowie b₁ bis b₄ Koeffizienten darstellen, und
der zu messende Muskel in dem bestimmten Körperteil ein Muskel, der sich auf Greifen bezieht, ein Rectus-Abdominis-Muskel, ein Quadrizeps-Femoris-Muskel oder ein Bizeps-Brachii-Muskel ist.

4. Vorrichtung nach einem der Ansprüche 1 bis 3, wobei die Rate der Änderung der Impedanz ein Wert ist, der ermittelt wird, indem die Impedanz des bestimmten Körperteils in einem gespannten Zustand durch die Impedanz des bestimmten Körperteils in einem entspannten Zustand dividiert wird.

5. Vorrichtung nach einem der Ansprüche 1 bis 3, wobei die Rate der Änderung der Impedanz ein Wert ist, der ermittelt wird, indem die Differenz zwischen der Impedanz des bestimmten Körperteils in einem gespannten Zustand und der Impedanz des bestimmten Körperteils in einem entspannten Zustand durch die Impedanz des bestimmten Körperteils in einem entspannten Zustand dividiert wird.

## Revendications

1. Dispositif de mesure de force musculaire à base d'impédance comprenant :
un moyen de direction d'état relâché du muscle,
un moyen de direction d'état contracté du muscle,
un moyen de mesure d'impédance,
un moyen de calcul de taux de changement d'impédance,
un moyen de stockage de formule de régression de force musculaire, et
un moyen de calcul de force musculaire,
dans lequel
le moyen de direction d'état relâché du muscle donne une direction selon laquelle un muscle dans une partie du corps spécifique doit être relâché,
le moyen de direction d'état contracté du muscle donne une direction selon laquelle le muscle dans la partie du corps spécifique doit être contracté,
le moyen de mesure d'impédance comporte un ensemble d'électrode pour être en contact avec la partie du corps spécifique et fournit un courant à la partie du corps spécifique et détecte une tension par l'utilisation de l'ensemble d'électrodes pour mesurer l'impédance du muscle relâché dans la partie du corps spécifique une fois que le moyen de direction d'état relâché du muscle a donné la direction et l'impédance du muscle contracté dans la partie du corps spécifique une fois que le moyen de direction d'état contracté du muscle donne la direction,
le moyen de calcul de taux de changement d'impédance calcule le taux de changement de l'impédance entre l'impédance du muscle relâché et l'impédance du muscle contracté qui sont mesurées par le moyen de mesure d'impédance,
le moyen de stockage de formule de régression de force musculaire stocke une formule de régression de force musculaire qui représente une corrélation entre le taux de changement dans l'impédance du muscle dans la partie du corps spécifique et la force musculaire de la partie de corps spécifique, et
le moyen de calcul de force musculaire calcule la force musculaire de la partie de corps spécifique en remplaçant le taux de changement de l'impédance qui est calculé par le moyen de calcul de taux de changement d'impédance dans la formule de régression de force musculaire stockée dans le moyen de stockage de formule de régression de force musculaire.

2. Dispositif selon la revendication 1, dans lequel l'ensemble d'électrodes du moyen de mesure d'impédance est en contact de manière mobile avec chacune des parties du corps spécifiques,
le moyen de stockage de formule de régression de force musculaire stocke une pluralité de formules de régression de force musculaire correspondant aux parties du corps spécifiques respectives et comprend un moyen de sélection/d'entrée de muscle pour sélectionner et entrer un muscle dans une partie du corps spécifique à mesurer et un moyen de sélection de formule de régression de force musculaire pour sélectionner une formule de régression de force musculaire correspondant à la partie du corps spécifique à mesurer qui est sélectionnée et entrée par le moyen de sélection/d'entrée de muscle parmi les formules de régression de force musculaire stockées dans le moyen de stockage de formule de régression de force musculaire, et le moyen de calcul de force musculaire calcule la force musculaire de la partie du corps spécifique en remplaçant le taux de changement de l'impédance qui est calculé par le moyen de calcul de taux de changement d'impédance dans la formule de régression de force musculaire sélectionnée par le moyen de sélection de formule de régression musculaire.

3. Dispositif selon la revendication 2, dans lequel les formules de régression de force musculaire sont des formules exprimées comme Y₁ = a₁X - b₁, Y₂ = a₂X - b₂, Y₃ = a₃X - b₃ et Y₄ = a₄X - b₄ dans lesquelles Y₁ représente la force musculaire d'un muscle associé à la saisie, Y₂ représente la force musculaire d'un muscle grand droit, Y₃ représente la force musculaire d'un muscle quadriceps crural, Y₄ représente la force musculaire d'un muscle biceps brachial, X représente le taux de changement de l'impédance et a₁ à a₄ et b₁ à b₄ représentent des coefficients, et
le muscle dans la partie du corps spécifique à mesurer est un muscle associé à la saisie, un muscle grand droit, un muscle quadriceps crural ou un muscle biceps brachial.

4. Dispositif selon l'une quelconque des revendications 1 à 3, dans lequel le taux de changement de l'impédance est une valeur obtenue en divisant l'impédance de la partie du corps spécifique dans un état contracté par l'impédance de la partie du corps spécifique dans un état relâché.

5. Dispositif selon l'une quelconque des revendications 1 à 3, dans lequel le taux de changement de l'impédance est une valeur obtenue en divisant la différence entre l'impédance de la partie du corps spécifique dans un état contracté et l'impédance de la partie du corps spécifique dans un état relâché par l'impédance de la partie du corps spécifique dans un état relâché.
